# EUROPÄISCHE PATENTSCHRIFT

(11) **EP 0 486 045 B1**
(45) Veröffentlichungstag und Bekanntmachung des Hinweises auf die Patenterteilung: **08.05.1996**
(21) Anmeldenummer: 91119522.0
(22) Anmeldetag: 15.11.1991
(51) Int. Cl.: C07C 57/30, A61K 31/205, C07C 229/26, C07C 59/64

(54) **Komplexe, enthaltend S-(+)-Phenyl-alkansäuren und alpha-Aminosäuren**
S(+)-Phenyl-alkanoic acids and alpha-amino acid containing complexes
Complexes contenant acides S(+)-phénylalkanoiques et l'acide alpha-amino

(30) Priorität: 15.11.1990 DE 4036458
(43) Veröffentlichungstag der Anmeldung: 20.05.1992
(73) Patentinhaber: MEDICEChem.-Pharm. Fabrik Pütter GmbH & Co. KG, D-58638 Iserlohn (DE)
(72) Erfinder: Paradies, Henrich Hasko, Prof. Dr. Dr., W-5860 Iserlohn (DE)
(74) Vertreter: Reinhard - Skuhra - Weise & Partner

(56) Entgegenhaltungen:
- EP-A- 0 267 321
- EP-A- 0 424 028
- DE-A- 2 419 317
- US-A- 4 279 926

## Beschreibung

Die vorliegende Erfindung betrifft wasserstoffbrücken-gebundene Komplexe mit einer Stöchiometrie von 1:1 aus S-(+)-Phenyl-alkansäuren und basischen a-Aminosäuren.

Verbindungen aus S-(+)-Phenyl-alkansäuren und a-Aminosäuren sind bereits bekannt. Es handelt sich dabei allerdings nicht um die speziellen erfindungsgemäßen Komplexe, sondern um Salze.

Lysinat-Salze, sowohl racemische (D, L) oder nur L-Salze basischer a-Aminosäuren sind u.a. bei Bruzzese et al. , US-Patentschrift 4,279,926 mit antiinflammatorischen Phenyl-alkansäuren, insbesondere mit (R,S)-Ibuprofen beschrieben worden. Sowohl die Europäische Patentanmeldung 0 267 321 von Loew et al, (1986), als auch die von Bruzzese et al. beschreiben pharmazeutische Formen, bestehend aus S-(+)-Ibuprofen und (D, L)-Lysin oder (R, S)-Ibuprofen mit (D, L) oder L-(+)-Lysin, als Salze, somit bestehend aus einem Anion und Kation.

Die Offenlegungsschrift DE 39 22441, A1, beschreibt pharmazeutische Zubereitungen von Ibuprofen und S-(+)-Ibuprofen mit einer niederen, neutralen oder sauren a-Aminosäure, welche bis zu 40 Gew.% zum Wirkstoff Ibuprofen (S-Ibuprofen) zugegeben werden. Die a-Aminosäuremenge kann sogar bis zu 60 % (g/g) zugegeben werden, sofern es sich um saure bzw. neutrale Aminosäuren handelt. Angaben zu Gewichts-Verhältnissen für basische a-Aminosäuren, noch deren Stereochemie, Wasserlöslichkeit, Stabilität und pharmakokinetisches Verhalten liegen nicht vor.

Die Druckschrift DE 3814887, C1, (1988), beschreibt weder Salze noch Komplexe mit D,L oder D, und L a-Aminosäuren, auch nicht mit basischen a-Aminosäuren, es wird lediglich eine Racemattrennung unter Zuhilfenahme von threo-1-p-Nitrophenyl-2-aminopropan-1,3-diol offenbart.

Die DE-A-24 19 317 beschreibt Lysinsalze von Ibuprofen. Die Bedeutung der Chiralität des Lysins und des Ibuprofens für günstige pharmakokinetische Eigenschaften wird nicht beschrieben. Weiterhin wird ausdrücklich auf das Vorliegen von Salzen hingewiesen; die erfindungsgemäßen 1:1 -Komplexe werden nicht beschrieben.

Die EP-A-0 424 028 beschreibt Komplexe aus L-(+)-Ibuprofen und einer L-Aminosäure. Die Anmeldung beansprucht die US-Priorität vom 17.10.1989 und wurde am 24.4.1991 offengelegt. Die Veröffentlichung ist somit Stand der Technik nach Art. 54(3) (4) EPÜ. Diese europäische Anmeldung beschreibt keine 1:1-Komplexe, sondern lediglich Salze zwischen Ibuprofen und einer a-Aminosäure.

Die EP 0 398 288, deren Miterfinder der Erfinder auch der vorliegenden Anmeldung ist, beansprucht die Priorität einer US-Anmeldung vom 16.5.1989 und wurde am 22.11.1990 offengelegt. Diese Anmeldung ist damit Stand der Technik nach Art. 84(3) (4) EPÜ. Die Anmeldung beschreibt zwar 1:1-Komplexe aus S-(+)-Ibuprofen und bestimmten a-Aminosäuren. Die pKa-Werte der Karboxylgruppen der S-(+)-Phenylalkansäuren und der basischen a-Aminosäuren werden jedoch nicht angegeben. Die vorliegende Anmeldung ist somit die erste Anmeldung im Sinne von Art. 87(1) EPÜ.

Eine Aufgabe der vorliegenden Erfindung ist es, neue Stoffe auf der Basis von S-(+)-Phenyl-alkansäuren und a-Aminosäuren zu schaffen und deren vorteilhafte Verwendung in pharmazeutischen Zubereitungen zu erarbeiten.

Diese Aufgabe wird erfindungsgemäß gelöst durch wasserstoffbrücken-gebundene Komplexe mit einer Stöchiometrie von 1:1 aus S-(+)-Phenyl-alkansäuren und basischen a-Aminosäuren, bei welchen die Komplex-Bindung auf Karboxylat-Karboxyl-Wechselwirkungen mit einem Proton-Switch der Form R₁-COOH...⁻OOC-R₂ 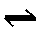 R₁-COO― ...HOOC-R₂ beruht, wobei R₁-COOH die S-(+)-Phenyl-alkansäuren und R_{z}-COOH die basischen a-Aminosäuren bedeuten, und wobei die pKa-Werte bezüglich der Karboxylgruppe der S-(+)-Phenyl-alkansäuren im Bereich von 3,5 - 3,9 und die pKa-Werte bezüglich der Karboxylgruppe der basischen a-Aminosäuren im Bereich von 1,9 - 2,9 liegen.

Vorzugsweise liegen die pKa-Werte bezüglich der Karboxylgruppe der basischen a-Aminosäuren im Bereich von 1,9-2,2.

Vorzugsweise sollen hier als S-(+)-Phenyl-alkansäuren auch S-(+)-Ibuprofen oder S-(+)-Naproxen verstanden und verwendet werden.

Vorzugsweise sollen hier als S-(+)-Phenyl-alkansäuren auch die nachfolgend beschriebenen Substanzen verstanden und verwendet werden. Diesen Verbindungen ist die untenstehende Strukturformel gemeinsam, 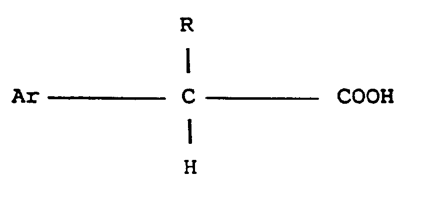 in welcher Rein Alkylrest ist, Ar vorzugsweise eine monozyklische, polyzyldische oder ortho-kondensierte polyzyklische aromatische Gruppe mit bis zu 12 Kohlenstoffatomen in dem aromatischen System, z. B. Phenyl, Diphenyl und Naphtyl. Die Substituenten dieser aromatischen Gruppen enthalten ein oder mehrere Halogenatome, C₁-C₄-Alkyle, Benzyl, Hydroxy, C₁-C₂-Alkoxy, Phenoxy und Benzoyl-Gruppen. Beispiele dieser substituierten Aryle sind: 4-Isobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl.

Vorzugsweise werden als basische a-Aminosäuren Lysin, Hydroxylysin, Arginin, Histidin oder Ornithin verwendet.

Vorzugsweise wird die D-Form der basischen a-Aminosäuren verwendet.

Erfindungsgemäß werden die erfindungsgemäßen Komplexe durch folgende Verfahrensschritte hergestellt:
a) zur Herstellung aus wäßrigem Medium (nur Wasser) oder schwach gepufferten wäßrigen Lösungen, die einen pH-Bereich umfassen zwischen pH 5.5-7.5 (20°C) bereitet man eine gepufferte, wäßrige Lösung, z. B. einen 0.01 M-0.001 M-K₂HPO₄/KH₂PO₄-Puffer pH 6.0-7.5 (20°C) vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird (normalerweise nach 20 Minuten) und alle S-(+)-Phenyl-alkansäure in Lösung gegangen ist;
c) anschließend wird der pH der Lösung auf pH 5.5-6.0 unter Zugabe von verdünnter Phosphorsäure (H₃P0₄) eingestellt (20°C) und danach wird die äquivalente (entsprechende) Menge der a-Aminosäure unter stetem Rühren eingetragen, wobei darauf zu achten ist, daß die neutralen a-Aminosäuren eingetragen werden, und nicht die salzsauren (HCI)-a-Aminosäuren!;
d) die Komplexbildung ist nach 20 Minuten beendet, worauf nach Abkühlen auf 0-4°C die Komplexe kristallin anfallen und über eine Glasnutsche oder einen Glasfilter (1 G4) von der Mutterlauge abgetrennt werden können;
e) alternativ zu Verfahrensschritt d) kann man die Ware Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert und aus Wasser/Ethanol (⁷⁰/₃₀ ^{V}/_{V}) oder aus Ethylacetat (100 %) umkristallisiert werden kann.

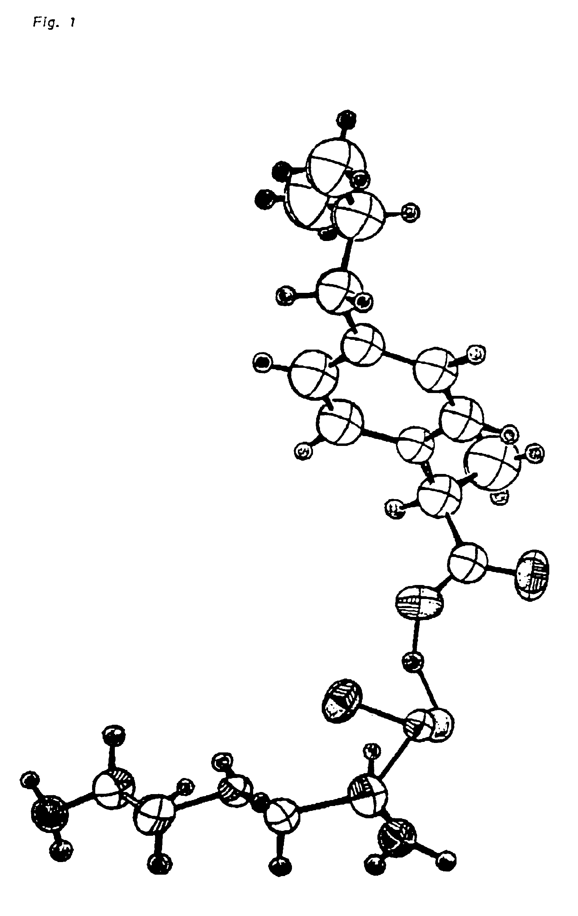

Bei den erfindungsgemäßen Stoffen handelt es sich nicht um eine Salzbildung zwischen einer sauren Gruppierung (Karboxylgruppe des Ibuprofens) und einem basischen Rest der a-Aminosäure (a-Amino, und/oder s-Amino-Gruppe oder Guanido-Gruppe des Lysins oder Arginins), sondern, wie die Röntgenstrukturanalyse und die FT-IR-Sprektren zeigen, um Karboxylat-Karboxyl-Wechselwirkungen, wobei sich beide Karboxyl-Reste der a-Aminosäure und z.B. des Ibuprofens, ein Proton teilen. D.h. der Komplex wird entsprechend der Röntgenstrukturanalyse durch eine Wasserstoffbrücke gebildet, ohne daß eine Beteiligung der a-Aminogruppe oder weiterer basischer Gruppen (Guanido-, ε-Amino- Gruppe) zu beobachten ist. (Fig. 1) Die Komplexe aus S-(+)-Ibuprofen und D-(-)-Lysin, oder aus S-(+)-Ibuprofen und L-(+)-Lysin kristallisieren in der chiralen Raumgruppe P2₁2₁2₁ als 1:1 Komplexe mit 4 Molekülen pro Einheitszelle. Innerhalb des Kristallgefüges sind die oben genannte und charakterisierte Wasserstoffbrückenbildung und die hydrophoben Kräfte des p-Isobutylphenylrestes die entscheidenden Wechselwirkungskräfte, die die kristalline Form (Fernordnung) zusammenhalten. Dies trifft auch für die Struktur in Lösung (H₂0) zu, da ausschließlich die pKa-Werte der a-Aminosäure bezüglich der Kaboxylgruppe, nämlich pKa = insbesondere 2,18 - 1,92, und der Phenyl-alkansäuren, nämlich pKa = 3,5 - 3,9, entscheidend sind. Die pKa-Werte der a-Amino-Gruppe der hier in Betracht kornenden a-Aminosäuren belaufen sich auf pKa = 8,9 - 9,10, der _{E}-Amino-Gruppe des Lysin z. B. auf pKa = 10,53, des Arginins (Guanido-Gruppe) ≈ 12,48. Daraus resultiert, daß die Aminogruppen der a-Aminosäuren bei der offenbarten Herstellung dieser Komplexe keine Rolle spielen, so daß auch keine Salzbildung vorliegen kann, nicht einmal ein intramolekulares lonenpaar, sondern vielmehr ein "Proton-Switch" der Form 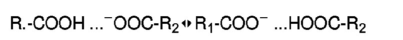 so daß der "Komplex" in Lösung neutral erscheint, ohne die Wasserlöslichkeit dieser Komplexe zu beeinflussen, wie es z.B. der Fall bei Alkali- und Erdalkalisalzen der Phenyl-alkansäuren, insbesondere bei Ibuprofen, da sie nur oberhalb pH>7,0 löslich - und dissoziert sind.

Die Dissoziaton dieser Alkali- oder Erdalkalisalze beeinflußt auch die Resorbierbarkeit nachteilig, so daß die therapeutischen Vorteile (z.B. keine Ausfällung des Ibuprofens im Magen, Resorption im gastro-intestinalen Bereich durch Ausbildung des Anions des Ibuprofens-Membranbarriere) der vorliegenden Komplexe erheblich sind, so daß die unerwünschten Nebenwirkungen bei gleicher Dosierung reduziert werden. FT-IR-Untersuchungen dieser Komplexe zeigen eindeutig, daß die a-Aminogruppe nicht komplex gebunden ist, da keine N-H-Streckschwingungen vorliegen, wohl aber nicht-wasserstoffbrücken-gebundene Karbonyl Gruppen bei 1680 - 1700 cm⁻¹. Ähnliche Ergebnisse wurden durch Ramanspektroskopische Untersuchungen erhalten, die weiterhin bestätigen, daß es sich bei der Struktur dieser hier beschriebenen Komplexe um Karboxyl-Karboxylat-Wechselwirkungen handelt.

Die erfindungsgemäßen Komplexe können vorteilhaft in pharmazeutischen Zubereitungen verwendet werden, enthaltend einen oder mehrere Komplexe und gegebenenfalls wahlweise zusätzlich physiologisch verträgliche gebräuchliche Streckungsmittel oder Träger.

Insbesondere vorteilhaft ist eine pharmazeutische Zubereitung auf der Grundlage von Phenyl-alkansäuren mit antiinflammatorischer, antipyretischer, antimikrobieller und analgetischer Wirkung, enthaltend einen Wirkstoffkomplex aus einer Phenyl-alkansäure und einer a-Aminosäure und ggf. zusätzlich übliche und physiologisch verträgliche Hilfsstoffe, bei der der Wirkstoffkomplex aus S-(+)-Phenyl-alkansäuren und basischen a-Aminosäuren besteht, vorzugsweise Da-Aminosäuren.

Insbesondere vorteilhaft ist eine pharmazeutische Zubereitung auf der Grundlage von Ibuprofen oder Naproxen mit antiinflammatorischer, antipyretischer, antimikrobieller und analgetischer Wirkung, enthaltend einen Wirkstoffkomplex aus einer Ibuprofen oder Naproxen und basischer a-Aminosäuren und ggf. zusätzlich übliche und physiologisch verträgliche Hilfsstoffe, bei der der Wirkstoffkomplex aus S-(+)-Ibuprofen oder S-(+)-Naproxen und einer basischen a-Aminosäure besteht und einen Gewichtsanteil 0,1 bis 90% (w/w) der Zusammensetzung ausmacht.

Insbesondere vorteilhaft ist eine pharmazeutische Zusammensetzung, die 50 bis 800 mg, vorzugsweise 100 bis 600 mg, insbesondere 100 bis 300 mg S-(+)-Ibuprofen bzw. S-(+)-Naproxen enthält.

Insbesondere vorteilhaft ist eine pharmazeutische Zubereitung, bei der die geeignete Dosis für die orale oder die parenterale Verabreichung im Bereich von 50 - 1200 mg pro Tag, normalerweise zwischen 100 und 800 mg pro Tag, vorzugsweise zwischen 200 und 600 mg S-(+)-Ibuprofen pro Tag liegt und daß die geeignete Dosis bei einer topischen Anwendung des Komplexes im Bereich von 10 - 200 mg pro Tag liegt.

Im folgenden wird die "pharmazeutisch aktive Verbindung" im weiteren Sinne als ein Komplex bezeichnet. In der medizinischen Anwendung kann diese pharmazeutisch aktive Verbindung oral, rektal, parenteral oder topisch angewendet werden, insbesondere jedoch oral oder topisch. Daher kann die therapeutische Zusammensetzung der vorliegenden Erfindung jede an sich bekannte pharmazeutische Zubereitung für orale, rektale, parenterale oder topische Anwendungen sein. Pharmazeutische gebräuchliche Träger, welche in solchen pharmazeutischen Zusammensetzungen angewendet werden, sind in der Pharmazie häufig beschrieben. Die Zusammensetzung dieser Erfindung kann 0,1 - 90% (w/w) der aktiven Verbindung entsprechen. Den Zusammensetzungen entsprechen normale, einheitliche Dosierungsformen. Diese Dosierungsformen enthalten 50 - 800 mg, vorzugsweise 100 - 600 mg oder 100 - 300 mg S-(+)-Ibuprofen.

Es werden orale Verabreichungsformen gemäß dieser Erfindung bevorzugt, wie z. B. Tabletten, Kapseln, Sirup und wäßrige oder ölige Suspensionen. Tabletten können z. B. hergestellt werden durch Mischen der aktiven Verbindung mit inerten Streckungsmitteln wie z. B. Calciumphosphat in Gegenwart eines aufschließenden Hilfsstoffes z. B. Stärke, oder Schmiermittels, z. B. Magnesiumstearat mit anschließender Tablettierung im normalen Herstellungssinne. Die Tabletten können in Form einer Retardformulierung der aktiven Verbindung nach bekannten Methoden hergestellt werden. Falls wünschenswert, können solche Tabletten nach entsprechend bekannten Methoden hergestellt werden, die sich im Magen nicht zersetzen; zum Beispiel unter Zuhilfenahme von Cellulose, Acetat, Phthalat. Entsprechend können Kapseln, z. B. Weich- und Hartgelatine-Kapseln hergestellt werden, welche die pharmazeutisch aktive Verbindung allein oder in Gegenwart von zugegebenen Hilfsstoffen enthalten. Diese Kapseln können mit üblicher pharmazeutischer Technologie, mit oder ohne magenresistenter Umhüllung, hergestellt werden. Andere Zusammensetzungen für orale Verabreichung schließen wäßrige Lösungen, die die aktive pharmazeutische Verbindung enthalten, in Gegenwart eines nicht toxischen Suspendierungsmittels, z. B. Carboxymethylcellulose und ölige Suspensionen, welche die aktive pharmazeutische Verbindung in Gegenwart eines Pflanzenöls.

Für die topische Anwendung der aktiven pharmazeutischen Verbindung können entsprechend dieser Erfindung pharmazeutische Formulierungen eingesetzt werden. Die pharmazeutisch aktive Verbindung wird in diesem Falle in einer pharmazeutisch geeigneten Creme, Salbe oder einem Gel dispergiert. Eine geeignete Creme kann z. B. dadurch hergestellt werden, daß die aktiv pharmazeutische Verbindung in einem topischen Träger wie z. B. leichtflüssiges Paraffin in einem wäßrigen Medium unter Zuhilfenahme von oberflächenaktiven Stoffen (Detergentien) dispergiert werden. Eine Salbe kann z. B. durch Mischen der pharmazeutisch aktiven Verbindung mit einem topischen Träger, wie z. B. Mineralöl oder Paraffin oder Bienenwachs hergestellt werden. Eine gelartige Formulierung kann durch Mischen einer aktiven pharmazeutischen Verbindung mit einem topischen Träger, z. B. Carbomer BP, in Gegenwart von Wasser hergestellt werden. Topisch anwendbare Zusammensetzungen können u.a. aus einer Matrix bestehen, die in der Lage ist, die aktive pharmazeutische Verbindung so zu dispergieren, daß diese durch ihren engen Kontakt mit der Haut transdermal verabreicht wird. Eine geeignete transdermale Zusammensetzung kann u. a. durch Mischen der pharmazeutisch aktiven Verbindung mit einem topischen Träger, wie vorne beschrieben, zusammen mit einem möglichen transdermalen Beschleuniger, z. B. Dimethylsulfoxid oder Propylenglykol, hergestellt werden.

Pharmazeutische Formulierungen entsprechend dieser Erfindung, die geeignet sind für die rektale Anwendung sind u. a. Suppositorien auf der Basis von Polyethylenglykol oder Kakaobutter.

Pharmazeutische Formulierungen zur parenteralen Anwendung beinhalten bekannte pharmazeutische Formulierungen z. B. sterile Suspensionen oder sterile Lösungen in einem geeigneten Lösungsmittel.

In einigen speziellen pharmazeutischen Formulierungen scheint es sinnvoll zu sein, die pharmazeutischen aktiven Verbindungen in einer Größe von kleinen Partikeln zu haben, z. B. kolloidale Lösungen oder partikuläre Suspensionen in der Größenordnung von 0,1 - 1 µm (Kolloidmühle).

Wenn wünschenswert, können entsprechend dieser Erfindung auch Zusammensetzungen mit anderen kompatiblen pharmazeutischen Wirkstoffen hergestellt werden.

Diese Komplexe entsprechend der Erfindung haben antiinflammatorische, antipyretische und interessante antimikrobielle Eigenschaften sowie analgetische Effekte. Diese Komplexe haben u. a. den Vorteil, daß sie nach oraler Verabreichung nach kürzerer Zeit wesentlich höhere Plasmaspiegel von S-(+)-Ibuprofen liefern als S-(+)-Ibuprofen in Form der freien Säure. Daher sind diese Komplexe in der Anwendung besonders wichtig zur Behandlung des akuten Schmerzes, wobei eine schnelle Anflutung mit sofortiger Schmerzbefreiung erzielt werden kann. Die Behandlung von Entzündungen und Schmerzen ist insbesondere wichtig bei Rheumapatienten, mit Indikationen wie primär chronische Polyarthritis, Arthritiden rheumatischen Ursprungs, Gelenkrheumatismus und Muskelrheumatismus mit ihren entsprechenden Schweregraden. Diese neuen Komplexe sind insbesondere wertvoll zur Schmerzentlastung, wie z. B. Kopfschmerzen, Dysmenorrhoe, postoperativer Schmerz, post-partum Schmerz und Schmerzen, die im Zusammenhang mit Grippe und Erkältungskrankheiten stehen.

Demgemäß beschreibt die Erfindung insbesondere einen anderen Aspekt zur Behandlung von Schmerz oder entzündlichem Fieber nach Verabreichung einer therapeutisch effektiven Dosis dieses Komplexes. Obwohl die genaue Dosis der pharmazeutisch aktiven Verbindung von einer Vielzahl von Parametern abhängig ist, z. B. Alter des Patienten, Zustand des Patienten, Anamnese und Compliance, liegt eine geeignete Dosis für sowohl orale als auch parenterale Verabreichungen von S-(+)-Ibuprofen-Komplex im Bereich von 50 - 1200 mg pro Tag, normalerweise zwischen 100 und 800 mg pro Tag, vorzugsweise zwischen 200 und 600 mg S-(+)-Ibuprofen pro Tag nach einmaliger oder mehrmaliger Verabreichung.

Bei einer topischen Anwendung dieses Komplexes liegt die entsprechende Dosis im Bereich von 10 - 200 mg pro Tag, im allgemeinen liegt die Dosis bei 20 - 100 mg pro Tag, je nach ärztlicher Anordnung.

Weitere Merkmale der Erfindung ergeben sich aus der folgenden Beschreibung von Ausführungsbeispielen:

### Beispiel 1:

### Herstellung des erfindungsgemäßen Komplexes:

146.2 g (1 mol) R-(+)-Lysin werden unter Rühren in 2 I demineralisiertem Wasser gelöst und zu dieser Lösung werden 206.2 g (1 mol) S-(+)-Ibuprofen zugefügt. Nach 10-minütigem Nachrühren wird die klare Lösung gefriergetrocknet. Die erhaltene feste Masse wird gemahlen, gesiebt und 3 Stunden bei 105°C nachgetrocknet. Ausbeute 345 - 349 g (98 - 99 % d. Th.), Schmp. 173.5 - 176.0°C.

### Beispiel 2:

### Herstellung einer erfindungsgemäßen Tablette:

### Zusammensetzung: 1 Tablette enthält

### wirksame Bestandteile

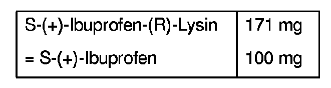

### nicht wirksame Bestandteile:

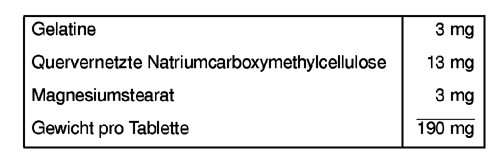

### Herstellung:

Die Gelatine wird zu 10 % in gereinigtem Wasser unter Erwärmen (max. 40°C) gelöst und dem Wirkstoff im Mischer bei geringer Mischleistung langsam zugefügt. Das erhaltene Granulat wird im Wirbelbett bei circa 40°C getrocknet und über eine Siebmaschine (Maschenweite 1,6 mm) abgesiebt. Das getrocknete Granulat wird mit Hilfe von Stempeln (Durchmesser 7,8 mm) zu Tabletten mit 190 mg Endgewicht verpreßt.

### Beispiel 3:

### Anwendungsbeispiel:

Maximale Plasmaspiegel von S-(+)-Ibuprofen (Cₘₐₓ in µg/ml) und diejenige Zeit, bei der maximale Plasmaspiegel von S-(+)-Ibuprofen erreicht wurden (tₘₐₓ , Std.) wurden gemessen bei vier gesunden männlichen Probanden nach Verabreichung von 150 mg S-(+)-Ibuprofen und 150 mg S-(+)-Ibuprofen-Komplex entsprechend dieser Erfindung, als amorphes Pulver verabreicht (siehe folgende Tabelle).

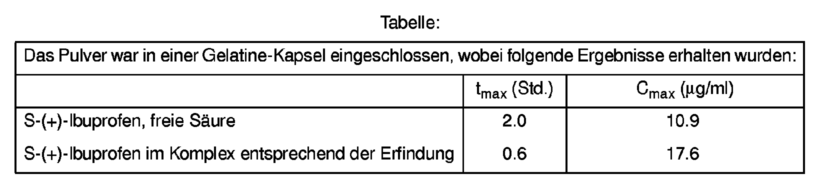

Vorteilhaft können auch erfindungsgemäß die erfindungsgemäßen Komplexe in pharmazeutischen Zubereitungen verwendet werden, wie sie in der deutschen Anmeldung DE 40 15 794.6 beschrieben sind. Solche isotropen Lösungen können hergestellt werden durch die folgenden Verfahrensschritte:
a.) Erwärmen des Trägers unter Rühren bis oberhalb des Schmelzpunktes, bis eine isotrope, transparente Flüssigkeit vorliegt;
b.) Messung der elektrischen Leitfähigkeit und der Viskosität bei der Temperatur des Schmelzpunktes, um das Vorliegen einer isotropen, transparenten Flüssigkeit zu gewährleisten;
c.) Bestimmung des refraktiven Index;
d.) Einstellen der gewünschten Konzentration des pharmazeutischen Wirkstoffs unter Beachtung des Molenbruches, der bei 37° C zwischen 0,001 und 0,67 liegen muß ;
e.) Eintragen des pharmazeutischen Wirkstoffes unter stetigem Rühren in das Lösungsmittel;
f.) Rühren des Ansatzes, bis der pharmazeutische Wirkstoff gelöst und eine transparente Lösung entstanden ist;
g.) Messung des differentiellen refraktiven Index-Inrementes [(An/Ac) T/P=konstant 1 zur Bestimmung der monomolekularen Lösung und/oder
h.) Kontrolle der nativen Konformation und der Monomolekularität des pharmazeutischen Wirkstoffes in der Lösung durch Messung des molaren Extinktionskoeffizienten im UV-Bereich und durch Aufnahme des Absorptionsspektrums sowie Feststellen der chiralen Konfiguration durch Messung im Polarimeter und/oder
i.) Messung der Trübung, um eine homogene Lösung sicherzustellen und/oder
k.) Messung der spezifischen Leitfähigkeit [(Ω)_{T,V=konstant}] Kontrolle der ionalen Konzentration in der isotropen Lösung;
1.) Abkühlen der klaren Lösung und Herstellung einer galenischen Formulierung ;
m.) weiteres Abkühlen der Lösung auf Raumtemperatur, bis die Lösung erstarrt ist.

Die hier erfindungsgemäß beschriebenen Komplexe, die nichtsalzartigen Charakter haben, bestehend z.B. aus S-(+)-Ibuprofen und einer basischen D-(-)-a-Amiosäure, die im Organismus in die entsprechende L-(+)-Aminosäure umgewandelt und verstoffwechselt wird, können in natürliche Membranen eingebaut werden, um
1.) die vorne genannte Resorbierbarkeit und
2.) um den genannten "Proton-Switch" nachzuweisen.

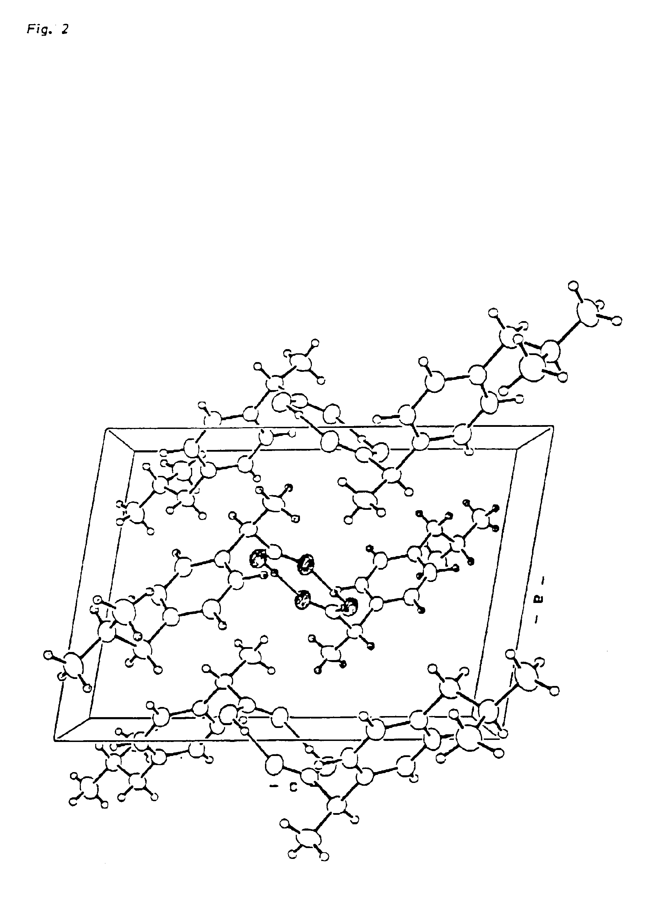

Die experimentelle Durchführung ist in den Literaturstellen, Paradies, H.H., Colloids Surf., (1985), 13, 263; Paradies, H.H., J- Phys. Chem. 90, 5956, (1986) beschrieben. Durch die Änderung der Fluoreszenz des Ibuprofens und des Ibuprofen-a-Aminosäure-Komplexes konnte de schelle Protontransfer nachgewiesen werden: kp = ₁,₇ × 10¹² M ⁻¹ sec ¹, der außerdem in der gleichen Größenordnung liegt für (R, S)-Ibuprofen, kp = 2,0 x 10¹² M⁻¹ sec⁻¹, S - (+) bzw. R-(-)-Ibuprofen kp = 2,3 x 1012 M⁻¹ sec-¹, hervorgerufen durch die Wechselwirkungen der Karboxylgruppen, die auch in den Kritallstrukturen wieder zu erkennen sind (Fig. 2). Salze bestehend aus Kation und Anion (Ibuprofen-Karboxylat) zeigen diese Effekte nicht! Der schnelle Wirkungseintritt einer Schmerzlinderung nach Verabreichung von S-(+)-Ibuprofen-D-(-) Lysinat, als auch die Pharmakokinetik sind die Folge dieser genannten Ergebnisse, welche - nun im folgenden dargelegt - in in vivo Experimenten an 24 Probanden belegt werden kann.

### Pharmakokinetik von S-(+)-Ibuprofen-D-(-)Lysinat

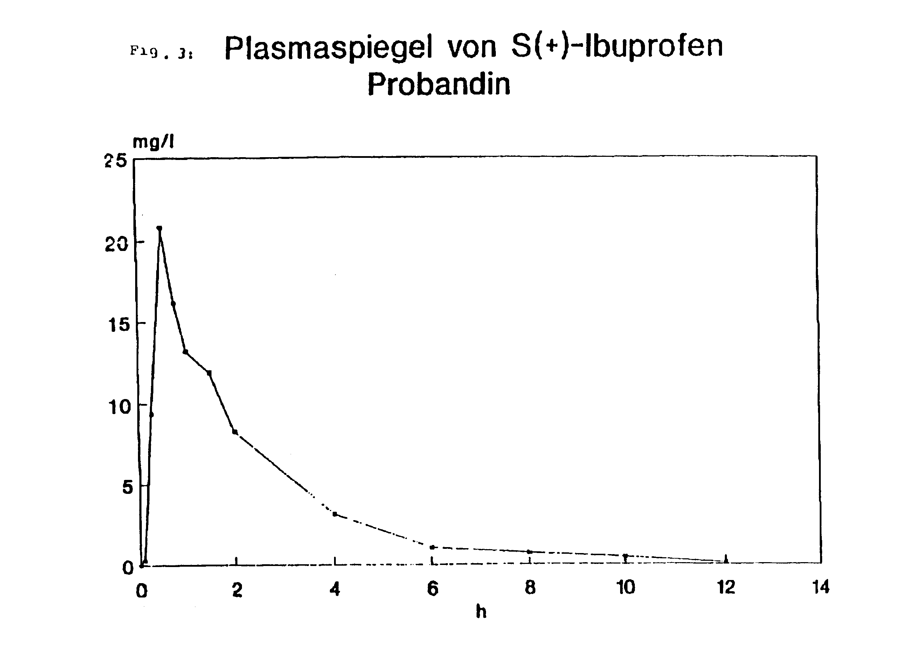

Die bei den 24 Probanden gemessenen Plasmaspiegelwerte von S-(+)-Ibuprofen sind in Tabelle 1 und 2 als Einzelwerte und Mittelwerte zusammengestellt, Fig. 3 zeigt eine repräsentative Plasmaspiegelkurve nach Verabreichung von S-(+)-Ibuprofen-D-Lysinat (200 mg S-(+)-Ibuprofen). Wie zu sehen, kam es in allen Fällen zu typischen Plasmaspiegelverläufen, wie sie nach oraler Einnahme eines Pharmakons zu erwarten sind. Hierbei folgte einer raschen Resorptionsphase mit Erreichen eines Maximalwertes ein rascher Abfall der Plasmaspiegel, in halblogarithmischer Darstellung als lineare Eliminationsphase erkennbar. Der für alle Probanden berechnete mittlere Plasmaspiegelverlauf mit dem unteren Bereich der einfachen Standardabweichung ist nach der Trapezregel berechnet worden und liegt als Mittelwert für alle Probandinnen bei 56,45 mg/1 x 12 h. Der maximale Plasmaspiegel Cₘₐₓ liegt bei 26,47 mg/1 und wird nach einer tₘₐₓ von 33 Minuten erreicht. Die Verzögerungszeit (lag-Time) der Resorption beträgt im Mittel 2,33 Minuten. Die Eliminationshalbwertzeit (T₁,₂p) beträgt für die Gesamtmittelwertskurve 107,4 Minuten (2,79 Stunden). In Tabelle 3 sind alle wichtigen pharmakokinetischen Kennwerte für alle Probanden bzw. die jeweiligen Untergruppen zusammengestellt.

### Pharmakodynamik von S-(+)-Ibuprofen-D-Lysinat

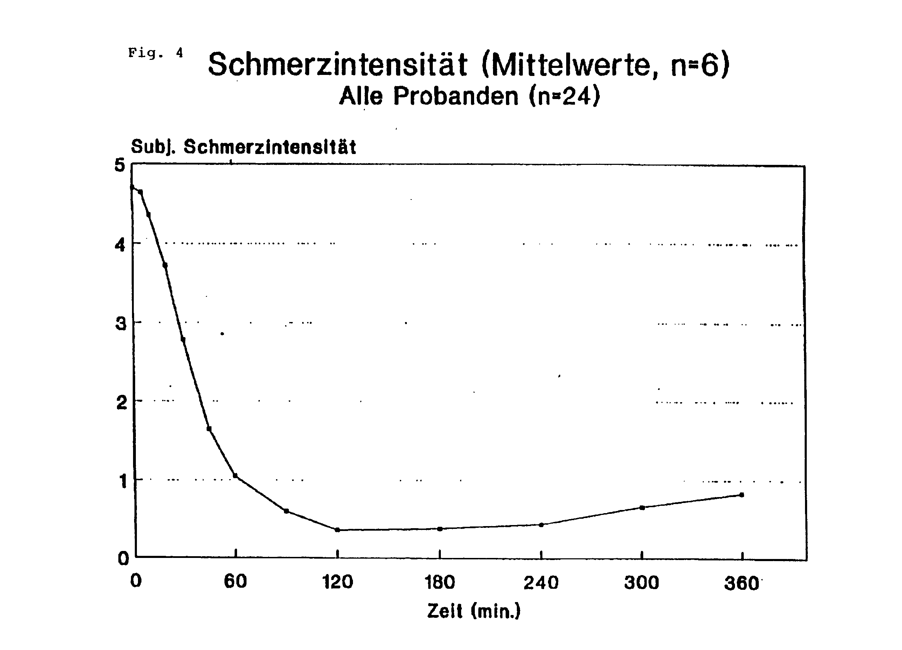

In einer monozentrienden Studie wurde die Pharmakodynamik bei primärer Dysmenorrhör an 24 Probandinnen untersucht. Die Mittelwerte aller Probandinnen als Auswertung einer Schmerzintensitätsskala sind in Tabelle 4 sowie Fig. 4 graphisch dargestellt. Die Schmerzintensität wurde anhand der visuellen Analogskala (einer von 0 - 10 offenen Skala) von den Probandinnen im Rahmen einer Selbstbeurteilung angegeben.

Wie aus Fig. 4 zu ersehen, betrug die mittlere Schmerzintensität zum Zeitpunkt Null 4,7, die analgetische Wirksamkeit setzte innerhalb von 5 Minuten ein und verminderte sich innerhalb der ersten 45 Minuten auf einen Wert von im Mittel 1,65. Die maximale analgetische Wirkung wurde nach 2 Stunden mit einem Wert von 0,36 erreicht. Anschließend stieg die Schmerzintensität bis auf einen Wert von 0,83 nach 6 Stunden wieder langsam an. Die Fig. 4 zeigt die Schmerzintensitätskurve der Probandinnen.

Unerwünschte Arzneimittelwirkungen traten bei keiner Teilnehmerin an der Studie auf. Ebenso waren alle laborchemischen Parameter sowie die Ergebnisse der ärztlichen Nachuntersuchung ohne pathologischen Befund. Es kann daraus geschlossen werden, daß die einmalige bzw. mehrmalige Einnahme von S-(+)-Ibuprofen-D-Lysinat (in dieser Studie bis zu 3 x täglich 200 mg an 3 Tagen) zu keinen unerwünschten Arzneimittelwirkungen geführt hat mit Ausnahme der bei einer Probandin aufgetretenen pseudoallergischen Reaktion.

Die relative orale Bioverfügbarkeit von S-(+)-Ibuprofen-D-Lysinat läßt sich durch Vergleich mit publizierten Literaturdaten abschätzen. Nach Einnahme von 200 mg S-(+)-Ibuprofen-D-Lysinat in Kapselform wurde eine mittlere AUC von 56,45 mg/1 x 12 h errechnet. Der Maximalspiegel betrug 26,47 mg/1 und wurde im Mittel nach 33,59 Minuten erreicht. Die Eliminationshalbwertzeit bei unseren weiblichen Probanden betrug 1,79 Stunden, die lag-Time 2,33 Minuten.

Ein pharmakokinetischer Vergleich von 150 mg S-(+)-Ibuprofen, freie Säure, Tablette mit S-(+)-Ibuprofen-C-Lysinat (150 mg S-(+)-Ibuprofen) ist zusammenfassend in Tabelle 5 dargelegt. Die schnelle Anflutungszeit (tₘₐₓ 0,6 ± 0,3 h, Lysinat), der hohe Cmax-Wert (17,6 ± 5,2) bei hoher AUC von 57,0 mg/ml x h zeigen die Überlegenheit des S-(+)-Ibuprofen-D-Lysinat-Komplexes über die Freie Säure des S-+)-Ibuprofens.

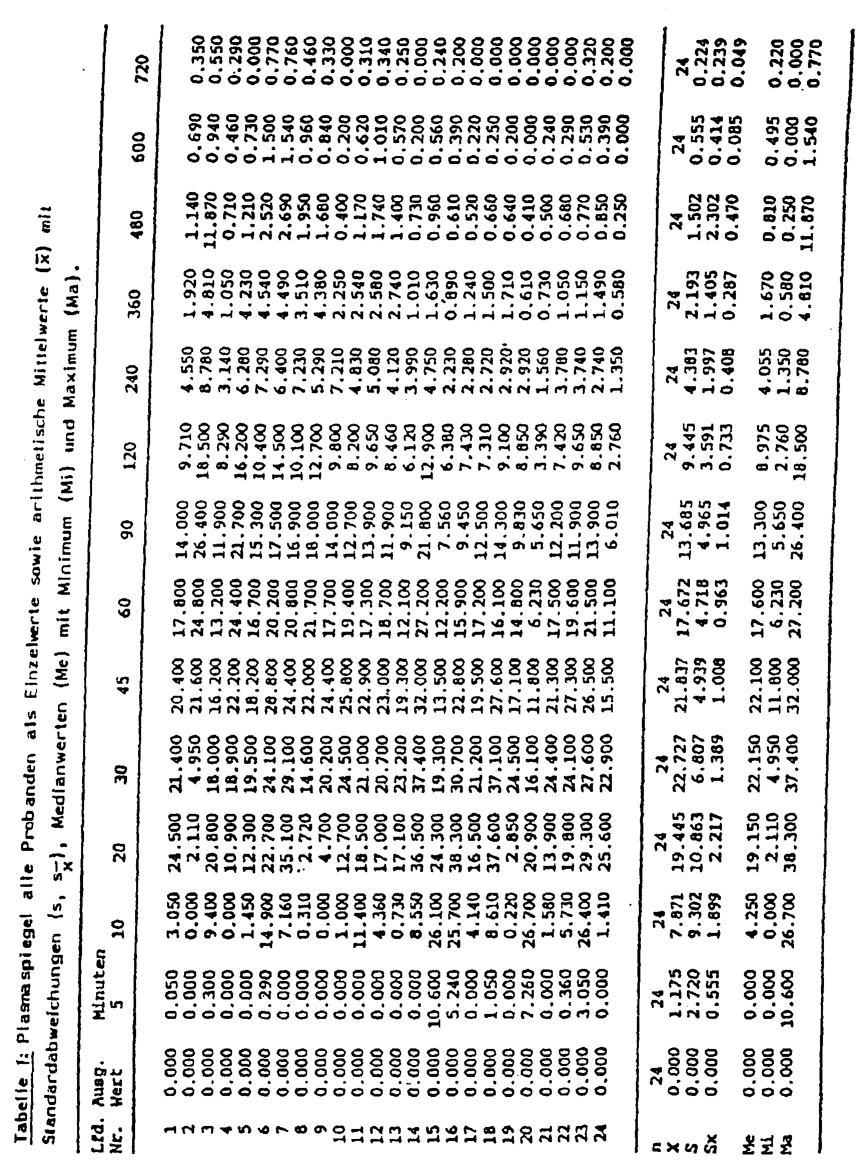

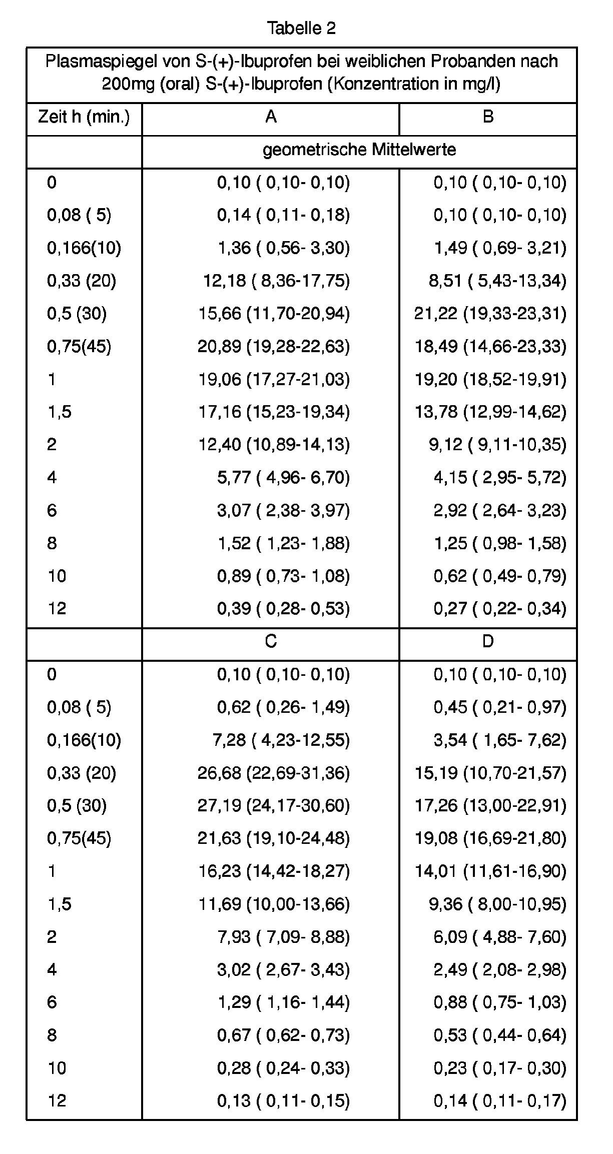

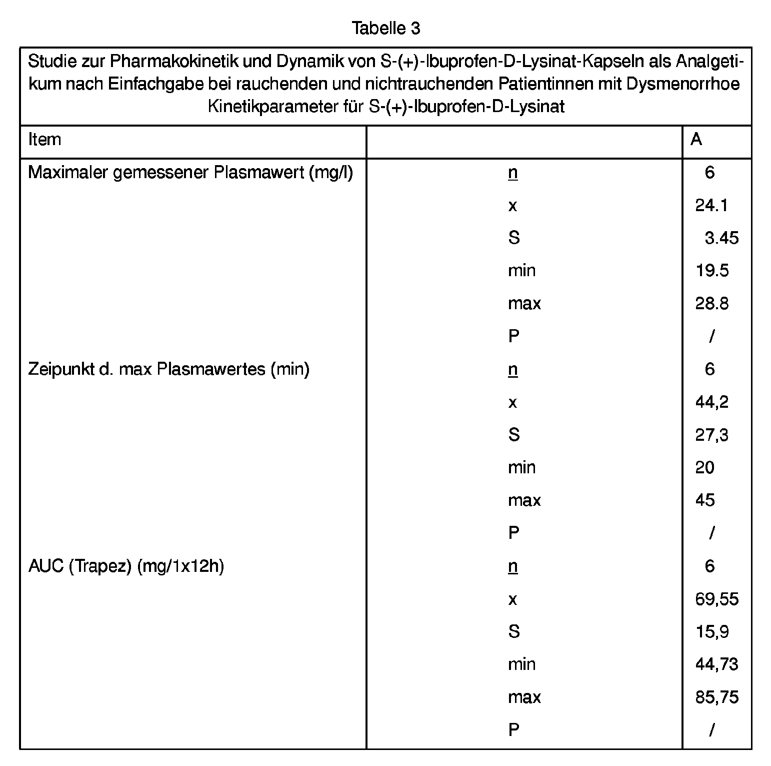

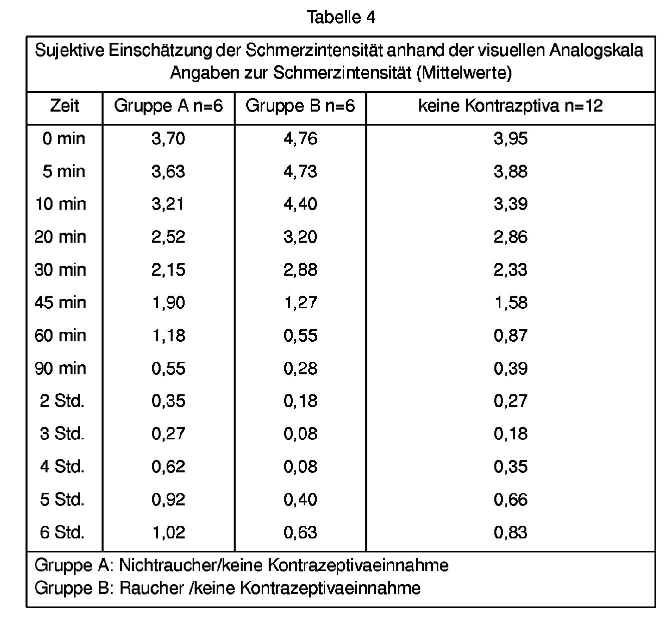

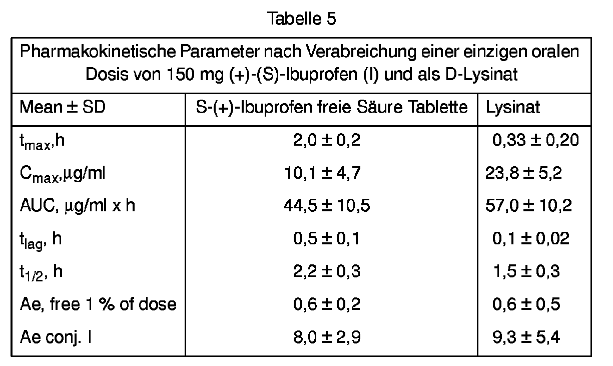

## Patentansprüche

1. Wasserstoffbrücken-gebundene Komplexe mit einer Stöchiometrie von 1:1 aus S-(+)-Phenyl-alkansäuren und basischen a-Aminosäuren, bei welchen die Komplex-Bindung auf Karboxylat-Karboxyl-Wechselwirkungen mit einem Proton-Switch der Form R₁-COOH...⁻OOC-R₂ 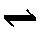 R₁-COO⁻...HOOC-R₂ beruht, wobei R₁-COOH die S-(+)-Phenyl-alkansäuren und R₂-COOH die basischen a-Aminosäuren bedeuten, und wobei die pKa-Werte bezüglich der Karboxylgruppe der S-(+)-Phenyl-alkansäuren im Bereich von 3,5 - 3,9 und die pKa-Werte bezüglich der Karboxylgruppe der basischen a-Aminosäuren im Bereich von 1,9 - 2,9 liegen.

2. Komplexe nach Anspruch 1,
dadurch gekennzeichnet,
daß die pKa-Werte bezüglich der Karboxylgruppe der basischen a-Aminosäuren im Bereich von 1,9 - 2,2 liegen.

3. Komplexe nach Anspruch 1 oder 2,
dadurch gekennzeichnet,
daß als S-(+)-Phenyl-alkansäuren S-(+)-Ibuprofen oder S-(+)-Naproxen verwendet werden.

4. Komplexe nach einem oder mehreren der vorhergehenden Ansprüche,
dadurch gekennzeichnet,
daß als basische a-Aminosäuren Lysin, Hydroxylysin, Arginin, Histidin oder Ornithin verwendet werden.

5. Komplexe nach einem oder mehreren der vorhergehenden Ansprüche,
dadurch gekennzeichnet,
daß die D-Form der basischen a-Aminosäuren verwendet wird.

6. Komplexe nach einem oder mehreren der vorhergehenden Ansprüche,
dadurch gekennzeichnet,
daß die S-(+)-Phenyl-alkansäuren von der Form 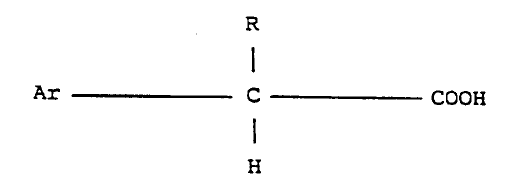 sind, in welcher R ein Alkylrest ist, Ar eine monozyklische, polyzyklische oder ortho-kondensierte polyzyklische aromatische Gruppe mit bis zu 12 Kohlenstoffatomen in dem aromatischen System, wie Phenyl, Diphenyl und Naphtyl ist und wobei die Substituenten dieser aromatischen Gruppen ein oder mehrere Halogenatome, C₁-C₄-Alkyle, Benzyl, Hydroxy, C₁-C₂-Alkoxy, Phenoxy und Benzoyl-Gruppen enthalten.

7. Komplexe nach einem oder mehreren der vorhergehenden Ansprüche,
dadurch gekennzeichnet,
daß die substituierten Aryle 4-lsobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl sind.

8. Verfahren zur Herstellung der Komplexe nach einem oder mehreren der vorhergehenden Ansprüche gekennzeichnet durch
folgende Verfahrensschritte:
a) zur Herstellung aus wäßrigem Medium (nur Wasser) oder schwach gepufferten wäßrigen Lösungen, die einen pH-Bereich umfassen zwischen pH 5.5-7.5 (20°C) bereitet man eine gepufferte, wäßrige Lösung vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird und alle S-(+)-Phenyl-alkansäure in Lösung gegangen ist;
c) anschließend wird der pH der Lösung auf pH 5.5-6.0 unter Zugabe von verdünnter Phosphorsäure (H₃P0₄) eingestellt (20°C) und danach wird die äquivalente (entsprechende) Menge der a-Aminosäure unter stetem Rühren eingetragen, wobei darauf zu achten ist, daß die neutralen a-Aminosäuren eingetragen werden, und nicht die salzsauren (HCI)-a-Aminosäuren!;
d) die Komplexbildung ist nach 20 Minuten beendet, worauf nach Abkühlen auf 0-4°C die Komplexe kristallin anfallen und über eine Glasnutsche oder einen Glasfilter (1 G4) von der Mutterlauge abgetrennt werden können;
e) alternativ zu Verfahrensschritt d) kann man die klare Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert und aus Wasser/Ethanol (⁷⁰/₃₀ ^{v}iᵥ) oder aus Ethylacetat (100 %) umkristallisiert werden kann.

9. Pharmazeutische Zubereitung, enthaltend einen oder mehrere Komplexe nach einem oder mehreren der vorhergehenden Ansprüche und gegebenenfalls wahlweise zusätzlich physiologisch verträgliche gebräuchliche Strekkungsmittel oder Träger.

10. Pharmazeutische Zubereitung nach Anspruch 9 auf der Grundlage von Phenyl-alkansäuren mit antiinflammatorischer, antipyretischer, antimikrobieller und analgetischer Wirkung, enthaltend einen Wirkstoffkomplex aus einer Phenyl-alkansäure und einer a-Aminosäure und ggf. zusätzlich übliche und physiologisch verträgliche Hilfsstoffe, dadurch gekennzeichnet, daß der Wirkstoffkomplex aus S-(+)-Phenyl-alkansäuren und basischen a-Aminosäuren besteht.

11. Pharmazeutische Zubereitung nach Anspruch 9 oder 10 auf der Grundlage von Ibuprofen oder Naproxen mit antiinflammatorischer, antipyretischer, antimikrobieller und analgetischer Wirkung, enthaltend einen Wirkstoffkomplex aus einer Ibuprofen oder Naproxen und basischer a-Aminosäuren und ggf. zusätzlich übliche und physiologisch verträgliche Hilfsstoffe,
dadurch gekennzeichnet,
daß der Wirkstoffkomplex aus S-(+)-Ibuprofen oder S-(+)-Naproxen und einer basischen a-Aminosäure besteht und einen Gewichtsanteil 0,1 bis 90% (w/w) der Zusammensetzung ausmacht.

12. Pharmazeutische Zusammensetzung nach einem oder mehreren der Ansprüche 9 bis 11,
dadurch gekennzeichnet,
daß sie 50 bis 800 mg S-(+)-Ibuprofen bzw. S-(+)-Naproxen enthält.

13. Pharmazeutische Zusammensetzung nach einem oder mehreren der Ansprüche 9 bis 12,
bestehend aus einem oder mehreren Wirkstoffkomplexen und einem wasserlöslichen Träger, der im Bereich zwischen 20 und 80°C erstarrt und wasserlöslich ist,
dadurch gekennzeichnet,
daß sie eine isotrope Lösung ist, wobei
a) der Wirkstoff in dem Träger monomolekular oder als Ion gelöst ist,
b) der Wirkstoff in seiner nativen Konformation und/oder seiner biologisch aktiven chiralen (enantiomeren) Konformation vorliegt.
c) der Wirkstoffe einen Molenbruch von 0,001 bis 0,67 bei 37°C aufweist,
d) der Träger bei Körpertemperatur schmelzflüssig, phaseneinheitlich und isotrop ist,
e) die isotrope Lösung, bestehend aus Träger und Wirkstoff, bei Raumtemperatur erstarrt,
f) die erstarrte Lösung kristallin oder nicht-kristallin ist oder sie den Wirkstoff kristallin enthält oder sie den Wirkstoff auskristallisieren kann,
g) die monomolekulare oder ionische Lösung einen osmotischen Druck hat und eine molare Gefrierpunkterniedrigung bewirkt,
h) der gelöste Wirkstoff innerhalb des Polymerelektrolyten einen temperaturabhängigen Diffusionskoeffizienten hat und eine temperaturabhängige spezifische Leitfähigkeit hat.

14. Pharmazeutische Zubereitung nach einem oder mehreren der Ansprüche 9 bis 13,
dadurch gekennzeichnet,
daß die geeignete Dosis für die orale oder die parenterale Verabreichung im Bereich von 50 - 1200 mg S-(+)-Ibuprofen pro Tag liegt und daß die geeignete Dosis bei einer topischen Anwendung des Komplexes im Bereich von 10 - 200 mg pro Tag liegt.

## Claims

1. Hydrogen-bridge-bound complexes having a stoichiometry of 1:1 comprising S(+)-phenyl alkane acids and basic a-amino acids in which the complex bond is based on carboxylate-carboxyl interactions with a proton switch of the form R₁-COOH...⁻OOC-R₂ 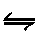 R₁-COO⁻ ... HOOC-R₂ where R₁-COOH denotes the S(+)-phenyl alkane acids and R_{z}-COOH the basic a-amino acids and the pKa values relating to the carboxyl group of the S(+)-phenyl alkane acids lie in the range of 3.5 -3.9 and the pKa values relating to the carboxyl group of the basic a-amino acids lie in the range of 1.9 - 2.9.

2. Complexes according to claim 1,
characterized in
that pKa values relating to the carboxyl group of the basic a-amino acids lie in the range of 1.9 - 2.2.

3. Complexes according to claim 1 or 2,
characterized in
that as S(+)-phenyl alkane acids S(+)-ibuprofen or S(+)-naproxen are used.

4. Complexes according to one or more of the preceding claims,
characterized in
that as basic a-amino acids lysine, hydroxy lysine, arginine, histidine or ornithine are used.

5. Complexes according to one or more of the preceding claims,
characterized in
that the D form of the basic a-amino acids is used.

6. Complexes according to one or more of the preceding claims,
characterized in
that the S(+)-phenyl alkane acids have a structure of the form 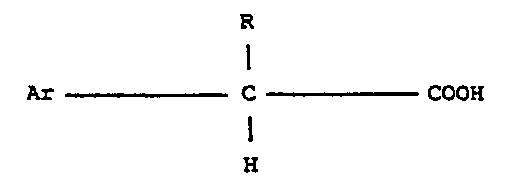 in which R is an alkyl residue, Ar is a monocyclic, polycyclic or ortho-condensed polycyclic aromatic group having up to twelve carbon atoms in the aromatic system, like phenyl, diphenyl, and naphthyl, and whereby the substituents of these aromatic groups comprise one or more halogen atoms, Ci-C4-a!ky!s, benzyl, hydroxy, C₁-C₂ alkoxy, phenoxy and benzoyl groups.

7. Complexes according to one or more of the preceding claims,
characterized in
that the substituted aryls are: 4-isobutyl-phenyl, 3-phenoxy-phenyl, 2-fluoro-4-diphenyl, 4'-fluoro-4-diphenyl, 6-methoxy-2-naphthyl, 5-chloro-6-methoxy-2-naphthyl and 5-bromo-6-methoxy-naphthyl, 4-chloro-phenyl, 4-difluorometh- oxy-phenyl, 6-hydroxy-2-naphthyl, and 5-bromo-6-hydroxy-2-naphthyl.

8. Method for the preparation of the complexes according to one or more of the preceding claims,
characterized by
the following method steps:
a) for the preparation from aqueous medium (only water) or weakly buffered aqueous solutions covering a pH range between pH 5.5 - 7.5 (20°C) a buffered aqueous solution is prepared and into it an equivalent amount S(+)-phenyl alkane acid is introduced with constant stirring;
b) the solution is heated with constant stirring to 40°C (water bath) until a clear transparent solution is obtained and all the S(+)-phenyl alkane acid has gone into solution;
c) thereafter the pH of the solution is adjusted to pH 5.5 - 6.0 by addition of diluted phosphoric acid (H₃P0₄) (20°C) and then the equivalent (corresponding) amount of the a-amino acid is introduced with constant stirring, ensuring that the neutral a-amino acids are introduced and not the hydrochloric acid (HCI)-a-amino acids!;
d) the complex formation is terminated after 20 minutes, whereupon after cooling to 0 - 4°C the complexes precipitate in crystalline form and can be separated from the mother liquor via a sintered glass funnel or glass filter (1G4);
e) alternatively to method step d) the clear solution can be reduced in a rotary evaporator (water bath temperature 25 - 30°C) in the water jet vacuum to half the volume, whereupon a colourless (amorphous) deposit forms which is filtered off via a 1 G4 glass filter and can be recrystallized from waterlethanol (70/30, v/v) or from ethyl acetate (100%).

9. Pharmaceutical preparation containing one or more complexes according to one or more of the preceding claims and possibly optionally additionally physiologically compatible extenders or carriers.

10. Pharmaceutical preparation according to claim 9 on the basis of phenyl alkane acids having anti-inflammatory, antipyretic, antimicrobial and analgesic effect, containing an active substance complex comprising a phenyl alkane acid and an a-amino acid and possibly additionally usual physiologically compatible auxiliary substances, characterized in that the active substance complex consists of S(+)-phenyl alkane acids and basic a-amino acids.

11. Pharmaceutical preparation according to claim 9 or 10 on the basis of ibuprofen or naproxen with anti-inflammatory, antipyretic, antimicrobial and analgesic effect, containing an active substance complex comprising an ibuprofen or naproxen and basic a-amino acids and possibly additionally usual physiologically compatible auxiliary substances, characterized in
that the active substance complex consists of S(+)-ibuprofen or (S+)-naproxen and a basic a-amino acid and represents an amount by weight of 0.1 to 90% (w/w) of the composition.

12. Pharmaceutical preparation according to one or more of claims 9 to 11,
characterized in
that it contains 50 to 800 mg S(+)-ibuprofen or S(+)-naproxen.

13. Pharmaceutical preparation according to one or more of claims 9 to 12, consisting of one or more active substance complexes and a water-soluble carrier which solidifies in the range between 20 and 80°C and is water-soluble, characterized in
that it is an isotropic solution, wherein
a) the active substance is dissolved in the carrier in monomolecular form or as ion,
b) the active substance is present in its native conformation and/or its biologically active chiral (enantiomeric) conformation,
c) the active substance has a molar fraction of 0.001 to 0.67 at 37°C,
d) the carrier is molten, phase-uniform and isotropic at body temperature,
e) the isotropic solution, consisting of carrier and active substance, solidifies at room temperature,
f) the solidified solution is crystalline or non-crystalline and contains the active substance in crystalline form or can crystallize the active substance out,
g) the monomolecular or ionic solution has an osmotic pressure and effects a molar freezing point reduction,
h) the dissolved active substance within the polymer electrolyte has a temperature-dependent diffusion coefficient and a temperature-dependent specific conductivity.

14. Pharmaceutical preparation according to one or more of claims 9 to 13,
characterized in
that the suitable dose for oral or parenteral administration is in the range of 50 - 1200 mg S(+)-ibuprofen daily and that the suitable doses for a topical administration of the complex lies in the range of 10 - 200 mg daily

## Revendications

1. Complexes liés par liaisons hydrogènes avec une stoechiométrie de 1:1 entre les acides S-(+)-phénylalcanoïques et des a-aminoacides basiques, dans lesquels la formation du complexe consiste en une interaction carboxylate- carboxyle avec un échange de proton de la forme
Rᵢ-COOH...'OOC-R₂ - Rₗ-COO-...HOOC-R₂, dans lesquels R₁-COOH représente les acides S-(+)-phénylalcanoï- ques et R_{z}-COOH représente des a-aminoacides et dans lesquels les pKa des groupes carboxyles des acides S-(+)-phénylalcanoïques sont compris entre 3,5 et 3,9 et les pKa des groupes carboxyles des a-aminoacides sont compris entre 1,9 et 2,9.

2. Complexes selon la revendication 1, caractérisés en ce que les pKa des groupes carboxyles des a-aminoacides basiques sont compris entre 1,9 et 2,2.

3. Complexes selon la revendication 1 ou 2, caractérisés en ce que,
le S-(+)-ibuprofène ou le S-(+)-naproxène sont utilisés à la place des acides S-(+)-phénylalcanoïques.

4. Complexes selon une ou plusieurs des revendications précédentes,
caractérisés en ce que,
la lysine, l'hydroxylysine, l'arginine, l'histidine ou l'ornithine sont utilisés comme a-aminoacides basiques.

5. Complexes selon une ou plusieurs des revendications précédentes,
caractérisés en ce que,
la forme D des a-aminoacides basiques est utilisée.

6. Complexes selon une ou plusieurs des revendications précédentes,
caractérisés en ce que,
les acides S-(+)-phénylalcanoïques sont de la forme 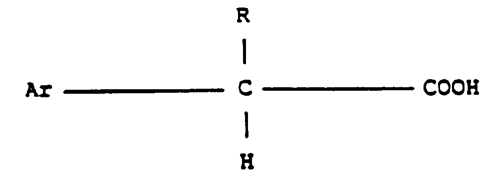 dans laquelle R est un résidu alkyle, Ar est un groupe aromatique monocyclique, polycyclique ou polycyclique orthocondensé avec jusqu'à 12 atomes de carbone dans le système aromatique, tel que le phényle, le diphényle et le naphtyle et dans lesquels les substituants de ces groupes aromatiques renferment un ou plusieurs atomes d'halogènes, des groupes C₁-C₄-alkyle, benzyle, hydroxy, C₁-C₂-alcoxy, phénoxy et benzoyl.

7. Complexes selon une ou plusieurs des revendications précédentes,
caractérisés en ce que,
les aryles substitués sont le 4-isobutyl-phényle, le 3-phénoxy-phényl, le 2-fluoro-4-diphényle, le 4'-fluoro-4-diphényle, le 6-méthoxy-2-naphtyle, le 5-chloro-6-méthoxy-2-naphtyle et le 5-bromo-6-méthoxy-naphty!e, !e 4-chioro-phényle, le 4-difluoro-méthoxy-phényle, le 6-hydroxy-2-naphtyle et le 5-bromo-6-hydroxy-2-naphtyle.

8. Procédé de fabrication des complexes selon une ou plusieurs des revendications
caractérisé par
les étapes de fabrication suivantes:
a) pour la fabrication en milieu aqueux (seulement de l'eau) ou en solutions aqueuses faiblement tamponnées dont le pH est compris entre 5,5 et 7,5 (20°C), on prépare une solution aqueuse tamponnée dans laquelle on rajoute une quantité équivalente d'acide S-(+)-phénylalcanoïque avec une agitation constante;
b) la solution est chauffée à 40°C (au bain-marie) avec une agitation constante jusqu'à l'obtention d'une solution claire, transparente et que tout l'acide S-(+)-phénylalcanoïque soit mis en solution;
c) ensuite le pH de la solution est ajusté à 5,5-6,0 par addition d'acide phosphorique dilué (H₃P0₄) (20°C) puis une quantité équivalente (correspondante) d'acide a-hydroxyalcanoïque est rajoutée avec une agitation constante.
d) la formation du complexe est réalisée au bout de 20 minutes après quoi le complexe est précipité en cristaux par refroidissement à 0-4°C et peut être séparé de la solution mère sur un entonnoir filtre en verre ou sur un filtre en verre (1G4);
e) alternativement à l'étape de fabrication d) la solution claire peut être réduite à la moitié de son volume dans un évaporateur rotatif (température du bain-marie 25-30°C) à trompe à vide, ce qui fait apparaître un précipité incolore (amorphe) qui est filtré sur un filtre en verre 1 G4 et peut être cristallisé dans un mélange eau/éthanol (70/30 V/V) ou dans de l'acétate d'éthyle (100%).

9. Composition pharmaceutique renfermant un ou plusieurs complexes selon une ou plusieurs des revendications précédentes et, au choix, un diluant ou un porteur courant physiologiquement compatibles.

10. Composition pharmaceutique selon la revendication 9, à base d'acide phénylalcanoïque ayant une action anti-inflammatoire, antipyrétique, antimicrobienne et analgésique, comprenant un complexe actif d'un acide phénylalcanoïque et d'un acide a-hydroxyalcanoïque et le cas échéant un agent auxiliaire supplémentaire, courant et physiologiquement compatible,
caractérisée en ce que,
le complexe actif se compose d'acides S-(+)-phénylalcanoïques et d'a-aminoacides basiques.

11. Composition pharmaceutique selon la revendication 9 ou 10 à base d'ibuprofène ou de naproxène ayant une action anti-inflammatoire, antipyrétique, antimicrobienne et analgésique, comprenant un complexe actif d'ibuprofène ou de naproxène et d'a-aminoacides basiques et, le cas échéant, un agent auxiliaire supplémentaire, courant et physiologiquement compatible,
caractérisée en ce que,
le complexe actif se compose de S-(+)-ibuprofène ou S-(+)-naproxène et d'un a-aminoacide basique et représente 0,1 à 90% en poids (p/p) de la préparation.

12. Composition pharmaceutique selon une ou plusieurs des revendications 9 à 11,
caractérisée en ce que,
elle renferme 50 à 800 mg de S-(+)-ibuprofène ou de S-(+)-naproxène.

13. Composition pharmaceutique selon une ou plusieurs des revendications 9 à 12,
composée d'un ou plusieurs complexes actifs et d'un porteur soluble dans l'eau qui fige dans le domaine compris entre 20 et 80°C et qui est soluble dans l'eau, caractérisée en ce que,
c'est une solution isotrope dans laquelle
a) l'agent actif est dissout sous forme d'ion ou sous forme monomoléculaire,
b) l'agent actif se présente sous sa conformation native et/ou sous sa conformation chirale biologiquement active (énantiomère),
c) l'agent actif présente une fraction de mole de 0,001 à 0,67 à 37°C,
d) l'agent porteur est liquéfié, en une seule phase et isotrope à la température corporelle,
e) la solution isotrope, composée d'un porteur et d'un agent actif se fige à température ambiante,
f) la solution figée est cristalline ou non cristalline ou elle renferme l'agent actif sous forme cristalline ou elle peut cristalliser l'agent actif,
g) la solution monomoléculaire ou ionique a une tension osmotique et abaisse le point de congélation molaire,
h) l'agent actif dissout dans le polymère électrolytique a un coefficient de diffusion dépendant de la température et une conductibilité spécifique dépendante de la température.

14. Composition pharmaceutique selon une ou plusieurs des revendications 9 à 13,
caractérisée en ce que,
la dose adaptée à l'administration orale ou parentérale est de l'ordre de 50 à 1200 mg par jour de S-(+)-ibuprofène et la dose adaptée à l'utilisation topique du complexe est de l'ordre de 10 à 200 mg par jour.
